# EUROPÄISCHE PATENTANMELDUNG

(11) **EP 2 281 800 A1**
(43) Veröffentlichungstag der Anmeldung: **09.02.2011**
(21) Anmeldenummer: 09014363.7
(22) Anmeldetag: 17.11.2009
(51) Int. Cl.: C07C 51/265, C07C 51/56, C07C 63/16, C07D 307/89

(54) **Verfahren zur Herstellung von Phthalsäure/Phthalsäureanhydrid**

(30) Priorität: 06.08.2009 DE 102009036295
(71) Anmelder: Lurgi GmbH, 60439 Frankfurt am Main (DE)
(72) Erfinder: Gutermuth, Thomas, Dr., 63477 Maintal (DE); Fiebig, Babett, Dr., 40597 Düsseldorf (DE); Kuchling, Thomas, 09600 Oberschöna (DE)

(57) **Zusammenfassung**

Verfahren zur kontinuierlichen Herstellung eines in einer aliphatischen Monocarbonsäure, vorzugsweise in Essigsäure, gelösten Gemisches aus Phthalsäure (PS) und Phtalsäureanhydrid (PSA) durch eine in Gegenwart eines Katalysators durchgeführte Oxidation mit Luftsauerstoff von in der Monocarbonsäure gelöstem o-Xylol, durchgeführt in mehreren Verfahrensstufen.

## Beschreibung

Die Erfindung betrifft ein Verfahren zur kontinuierlichen Herstellung eines in einer aliphatischen Monocarbonsäure, vorzugsweise in Essigsäure, gelösten Gemisches aus Phthalsäure (PS) und Phthalsäureanhydrid (PSA) durch eine in Gegenwart eines Katalysators durchgeführte Oxidation mit Luftsauerstoff von in der Monocarbonsäure gelöstem o-Xylol.

PSA ist ein wichtiges chemisches Zwischenprodukt zur Erzeugung von Phthalatweichmachern, ungesättigten Polyestern und Alkydharzen für die Oberflächentechnik.

Die großtechnische Herstellung von PSA erfolgt üblicherweise durch Gasphasenoxidation von o-Xylol mit Luftsauerstoff in einer Katalysatorschüttung.

Der chemische Ablauf der Herstellung von PS/PSA aus o-Xylol in der Gasphase ist in Ullmann's Encyclopedia of Industrial Chemistry, Sixth Edition, Electronic Release, Phthalic Acid and Derivates, Chapt. 2.4 dargestellt.

Neben der Gasphasenoxidation gibt es seit langem Verfahrensalternativen, bei denen die Oxidationsreaktion in der flüssigen Phase, entweder unverdünnt oder gelöst in einer aliphatischen Monocarbonsäure, üblicherweise Essigsäure, durchgeführt wird. Die in flüssiger Phase ablaufenden Verfahren haben gegenüber den in Gasphase ablaufenden den Vorteil, dass sie bei tieferen und damit leichter beherrschbaren Temperaturen ablaufen. Eine möglichst genaue Steuerung der Reaktionstemperatur ist wichtig für die Erzielung einer hohen Rohstoffausbeute. Besonders vorteilhaft ist die Durchführung in verdünnter flüssiger Phase, weil dabei die Möglichkeit gegeben ist, die in erheblichem Umfang anfallende Reaktionsenthalpie der Oxidationsreaktionen durch Verdampfung des Lösungsmittels abzuführen.

Schon in der DE 1 267 677A ist ein entsprechendes Verfahren zur Herstellung von Phthalsäure und Phthalsäureanhydrid durch Oxidation mit Luftsauerstoff von in Essigsäure gelöstem o-Xylol beschrieben. Dabei wird die Durchführung des Verfahrens in einer aus drei Reaktoren bestehenden Kaskade vorgeschlagen. Allerdings liefert diese Schrift keine Hinweise zur Lösung der im Folgenden beschriebenen Probleme, die bei der Durchführung dieses chemischen Prozesses auftreten.

Die Probleme sind:
a) Eigene Untersuchungen haben ergeben, dass die bei der Oxidation von o-Xylol zu PS/PSA auftretenden Zwischenprodukte 2-Methylbenzaldehyd, 2-Methylbenzoesäure und Phthalid so stabil sind, dass sie, in Anwesenheit von o-Xylol, nur sehr schwer zu oxidieren sind. Dies führt bei einem kontinuierlichen Herstellungsverfahren, bei dem ständig o-Xylol in den Reaktionsraum nachgespeist wird dazu, dass das Endprodukt PS/PSA mit einem hohen Anteil an Zwischenprodukten verunreinigt ist. Diese Zwischenprodukte erhöhen mit zunehmendem Gehalt den Reinigungsaufwand des Produktes PS/PSA und bedeuten eine Verschlechterung der Rohstoffausbeute.
b) PS und PSA sind nicht resistent gegen Sauerstoff, sodass, um Verluste zu vermeiden, deren Verweilzeit im Reaktor unter Sauerstoffangriff möglichst kurz gehalten werden muss.
c) Die an der Oxidation beteiligten Stoffe sind sehr korrosiv, sodass für die Reaktoren sehr teure Konstruktionsmaterialien verwendet werden müssen. Die Größe der Reaktoren beeinflusst daher stark die Wirtschaftlichkeit des Verfahrens. Auch aus diesem Grund muss die Verweilzeit kurz gehalten werden.

Aufgabe der Erfindung ist es daher, ein Verfahren zur Verfügung zu stellen, das in der Lage ist, die bei der Oxidation anfallenden Zwischenprodukte möglichst vollständig in PS und PSA umzuwandeln und dabei mit möglichst wenigen und möglichst kleinen Reaktoren auszukommen.

Die Aufgabe wird erfindungsgemäß dadurch gelöst, dass das Verfahren in mehreren, vorzugsweise in zwei, hintereinander geschalteten Reaktionsstufen abläuft, wobei in dem der letzten Reaktionsstufe zugeführten Produktstrom das Molverhältnis von o-Xylol zu den aus 2-Methylbenzaldehyd, 2-Methylbenzoesäure und Phthalid gebildeten Zwischenprodukten unter 1:40 liegt. Eine besonders vorteilhafte Ausgestaltung der Erfindung ist dann gegeben, wenn in dem der letzten Reaktionsstufe zugeführten Produktstrom das Molverhältnis von o-Xylol zur Summe aus Phthalsäure und Phthalsäureanhydrid über 1:65 liegt.

Diese Zusammensetzung ist die Voraussetzung dafür, dass im letzten Reaktor die Zwischenprodukte 2-Methylbenzaldehyd, 2-Methylbenzoesäure und Phthalid soweit zu PS/PSA umgesetzt werden können, dass der diesen Reaktor verlassende Stoffstrom nicht mehr Zwischenprodukte enthält als der in den ersten Reaktor eintretende Stoffstrom.

In einer vorteilhaften Ausgestaltung der Erfindung wird die positive Reaktionsenthalpie aus den Reaktionsstufen ganz oder teilweise durch Verdampfung eines Teils der aliphatischen Monocarbonsäure abgeführt.

Es ist vorteilhaft, wenn der verwendete Katalysator aus Salzen der Elemente Cobalt, Mangan und Brom besteht und besonders vorteilhaft, wenn er zusätzlich aus Salzen des Elements Zirkonium besteht. Der Katalysator wird dem o-Xylol/Essigsäure-Gemisch vor dem Eintritt in den ersten Oxidationsreaktor zugegeben.

Weiterhin ist es vorteilhaft, wenn in dem dem ersten Reaktor zugeführten Stoffstrom das Molverhältnis von o-Xylol zu Essigsäure zwischen 1: 16 und 1: 19, bevorzugt zwischen 1:17 und 1:18 liegt. Bei diesem Mischungsverhältnis verläuft die Reaktion mit einer verlangsamten, für die Abführung der Reaktionsenthalpie und der damit verbundenen Einstellung der Reaktionstemperatur optimalen Geschwindigkeit, die gleichzeitig aber schnell genug ist, um wirtschaftlich zu sein.

Ebenso zu diesem Zweck wird die Reaktionstemperatur in der ersten Verfahrensstufe zwischen 125 und 135°C und in der zweiten Verfahrensstufe zwischen 105 und 125°C eingestellt.

Reaktoren für die Durchführung des Verfahrens müssen eine gute Durchmischung des gasförmigen Sauerstoffs mit der flüssigen Phase gewährleisten. Bei den Reaktoren handelt es sich um Druckbehälter zur Aufnahme des flüssigen Reaktionsgemisches. Sie sind im Bodenbereich mit einer Vorrichtung zur gleichmäßigen Verteilung von gasförmigen Sauerstoff, bevorzugt Luftsauerstoff, in der Flüssigkeit ausgestattet. Die Behälter können mit Rührwerken ausgestattet sein. Bevorzugt handelt es sich um Blasensäulenreaktoren, bei denen die Flüssigkeit und der Luftsauerstoff am Boden eintreten und die Flüssigkeit am oberen Ende austritt. Die durch die Oxidationsreaktionen freigesetzte Reaktionsenthalpie führt zu einem Temperaturanstieg der Flüssigkeit zu dem sich ein entsprechender Dampfdruck im Behälter einstellt. Jeder Reaktor ist mit einer Druckregeleinrichtung ausgestattet. Überschüssige Reaktionsenthalpie wird durch Verdampfung des Lösungsmittels, in der Regel Essigsäure, abgeführt.

Blasensäulenreaktoren sind insbesondere geeignet, da bei ihnen, im Vergleich zu einem Rührkessel, eine Vermischung zwischen den Ausgangs-, Zwischen-und Endprodukten in geringerem Umfang erfolgt, sondern eine örtliche Trennung zwischen dem am Boden eintretenden Eingangsstrom der Flüssigkeit und dem am oberen Ende austretenden Ausgangsstrom besteht. Dies kommt der Umsetzung der Zwischenprodukte entgegen, da im oberen Reaktorbereich der Gehalt an o-Xylol abnimmt.

Um diesen Effekt zu unterstützen ist bei einer besonders vorteilhaften Ausgestaltung der Erfindung das Verhältnis von Höhe zu Durchmesser der Blasensäulenreaktoren größer als 9 : 1.

Die Einhaltung der vorgenannten Bedingungen ermöglichen die Durchführung des erfindungsgemäßen Verfahrens mit einer höheren Umsetzung der Reaktanden zum Endprodukt und einer kürzeren Verweilzeit, während der sie dem Kontakt mit Sauerstoff ausgesetzt sind, als wenn die Herstellung von PS/PSA aus o-Xylol einstufig, in einem Reaktionsraum durchgeführt würde. Die Verkürzung der Verweilzeit ermöglicht kleinere Reaktorabmessungen und verringert die Verluste an PS/PSA durch den Sauerstoffangriff.

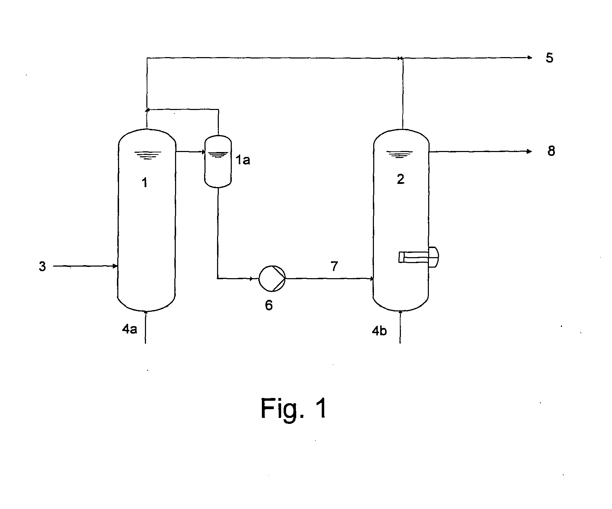

Das erfindungsgemäße Verfahren wird an Hand des Beispiels 1 mit der Zeichnung Fig. 1 und der Stoffstromtabelle erläutert.

### Beispiel 1:

Das Verfahren läuft in zwei, hintereinander geschalteten, Reaktoren 1 und 2 ab. Reaktor 2 ist mit einem Erhitzer ausgestattet. Der Behälter 1a nimmt die von Reaktor 1 kommende Flüssigkeit auf und dient als Vorlagebehälter für die Pumpe 6, die die Flüssigkeit in Reaktor 2 einspeist. Mit Stoffstrom 3 wird der Rohstoff der Oxidationsreaktion, o-Xylol, in den ersten Reaktor 1 eingegeben. Über die Stoffstromleitung 4a bzw. 4b wird Luftsauerstoff in die Reaktoren eingeleitet. Über die Stoffstromleitung 5 werden die Abgase aus den Reaktoren 1 und 2 sowie dem Behälter 1 a abgeführt. Weil es vor dem Einsatz in Reaktor 1 bereits, außerhalb dieses Verfahrens, als Extraktionsmittel zur Entfernung von Zwischenprodukten aus PS/PSA verwendet wurde, enthält es in der Summe 1,9 mol-% 2-Methylbenzaldehyd, 2-Methylbenzoesäure und Phthalid.

O-Xylol ist dabei in einem Molverhältnis von 1:17,2 in Essigsäure gelöst. Dem Stoffstrom 3 sind als Katalysator Salze der Elemente Cobalt, Mangan, Brom und Zirkonium zugegeben. Das molare Verhältnis von o-Xylol zu Cobalt beträgt 1: 0,01. Die molaren Verhältnisse der Katalysatorkomponenten Co:Mn:Br:Zr untereinander betragen 1:1:2,5:0,01. Das Katalysatorgemisch wird pulverförmig in Essigsäure eingemischt, bevor die Essigsäure mit o-Xylol zu Stoffstrom 3 vermischt wird. Stoffstrom 4 stellt den für die Oxidationsreaktion verwendeten Luftsauerstoff dar. Stoffstrom 5 stellt den Abgasstrom dar, der hauptsächlich aus verdampfter Essigsäure, aus bei der Oxidation entstandenem Wasserdampf und der mit Stoffstrom 4 eingetragenen Luft besteht.

In Reaktor 1 beträgt die Reaktionstemperatur 130°C, dazu wird ein Druck von ungefähr 5 bar eingestellt. Die mittlere Verweilzeit in Reaktor 1 beträgt 75 min. Temperatur und Verweilzeit sind so gewählt, dass in Stoffstrom 7, der den Reaktor 1 verlässt und über eine Pumpe 6 in Reaktor 2 gegeben wird, das Molverhältnis von o-Xylol zu den aus 2-Methylbenzaldehyd, 2-Methylbenzoesäure und Phthalid gebildeten Zwischenprodukten 1:42 und das Molverhältnis von o-Xylol zur Summe aus PS/PSA 1:62 beträgt. In Reaktor 2 wird eine Reaktionstemperatur von 110°C und ebenfal ls eine mittlere Verweilzeit von 75 Minuten eingestellt. Das Verhältnis von Durchmesser zu Höhe bei Reaktor 1 und 2 beträgt 1:10. Über Leitung 8 verlässt das Verfahrensprodukt PS/PSA, gelöst in Essigsäure, die Anlage, um in weiteren, an sich bekannten Verfahren zu reinem, kristallinem Phthalsäureanhydrid verarbeitet zu werden. Stoffstromtabelle:

| **Stoffstrom** | | **3** | **4a** | **4b** | **5** | **7** | **8** |
|---|---|---|---|---|---|---|---|
| o-Xylol | mol/Std. | 9,70 | | | 0,28 | 0,09 | |
| Mbs *) | mol/Std. | 0,05 | | | | 2,83 | 0,05 |
| Phthalid | mol/Std. | 0,14 | | | | 0,94 | 0,14 |
| H₂O | mol/Std. | | | | 13,49 | | 11,49 |
| PS+PSA | mol/Std. | | | | | 5,57 | 9,41 |
| N₂ | mol/Std. | | 93,3 | 46,6 | 139,19 | | |
| O₂ | mol/Std. | | 24,6 | 12,3 | 8,94 | | |
| Essigsäure | mol/Std. | 167,00 | | | 88,51 | | 78,49 |

| | | | | | | | |
|---|---|---|---|---|---|---|---|
| *) 2-Methylbenzaldehyd, 2-Methylbenzoesäure | | | | | | | |

## Patentansprüche

1. Verfahren zur kontinuierlichen Herstellung eines in einer aliphatischen Monocarbonsäure, vorzugsweise in Essigsäure, gelösten Gemisches aus Phthalsäure und Phtalsäureanhydrid durch eine in Gegenwart eines Katalysators durchgeführte Oxidation mit Luftsauerstoff von in der Monocarbonsäure gelöstem o-Xylol, **gekennzeichnet durch** mehrere Reaktionsstufen, wobei in dem der letzten Reaktionsstufe zugeführten Produktstrom (7) das Molverhältnis von o-Xylol zu den aus 2-Methylbenzaldehyd, 2-Methylbenzoesäure und Phthalid gebildeten Zwischenprodukten unter 1:40 liegt.

2. Verfahren nach Anspruch 1, **dadurch gekennzeichnet, dass** in dem der letzten Reaktionsstufe zugeführten Produktstrom (7) das Molverhältnis von o-Xylol zur Summe aus Phthalsäure und Phthalsäureanhydrid über 1:65 liegt.

3. Verfahren nach Anspruch 1, **dadurch gekennzeichnet, dass** das Verfahren in zwei Reaktionsstufen durchgeführt wird.

4. Verfahren nach einem der vorhergehenden Ansprüche, **dadurch gekennzeichnet, dass** die positive Reaktionsenthalpie aus den Reaktionsstufen ganz oder teilweise durch Verdampfung eines Teils der aliphatischen Monocarbonsäure abgeführt wird.

5. Verfahren nach Anspruch 1, **dadurch gekennzeichnet, dass** der Katalysator aus Salzen der Elemente Cobalt, Mangan und Brom besteht.

6. Verfahren nach Anspruch 1, **dadurch gekennzeichnet, dass** der Katalysator aus einem Gemisch aus Salzen der Elemente Cobalt, Mangan, Brom und Zirkonium besteht.

7. Verfahren nach einem der vorhergehenden Ansprüche, **dadurch gekennzeichnet, dass** in dem dem ersten Reaktor zugeführten Stoffstrom 3 das Molverhältnis von o-Xylol zu Essigsäure zwischen 1: 16 und 1: 19, bevorzugt zwischen 1:17 und 1:18 liegt.

8. Verfahren nach einem der vorhergehenden Ansprüche, **dadurch gekennzeichnet, dass** die Reaktionstemperatur in der ersten Verfahrensstufe zwischen 125 und 135°C und in der zweiten Verfahren sstufe zwischen 105 und 125°C eingestellt wird.

9. Verfahren nach einem der vorhergehenden Ansprüche, **dadurch gekennzeichnet, dass** beide Verfahrensstufen in jeweils einem Blasensäulenreaktor durchgeführt werden.

10. Verfahren nach Anspruch 8, **dadurch gekennzeichnet, dass** das Verhältnis von Höhe zu Durchmesser der Blasensäulenreaktoren größer als 9 : 1 ist.
